# EUROPÄISCHE PATENTSCHRIFT

(11) **EP 0 784 465 B1**
(45) Veröffentlichungstag und Bekanntmachung des Hinweises auf die Patenterteilung: **16.06.1999**
(21) Anmeldenummer: 95934113.2
(22) Anmeldetag: 25.09.1995
(51) Int. Cl.: A61K 7/06, A61K 7/48, A61K 7/50

(54) **KOSMETISCHE MITTEL ENTHALTEND FETTSÄURE-N-ALKYLGLUCAMIDE UND SILICONVERBINDUNGEN**
COSMETIC AGENTS CONTAINING FATTY ACID-N-ALKYL GLUCAMIDE AND SILICON COMPOUNDS
PRODUITS COSMETIQUES CONTENANT DU N-ALKYLGLUCAMIDE D'ACIDE GRAS ET DES COMPOSES DE SILICONE

(30) Priorität: 04.10.1994 DE 4435383
(43) Veröffentlichungstag der Anmeldung: 23.07.1997
(73) Patentinhaber: Henkel Kommanditgesellschaft auf Aktien, 40191 Düsseldorf (DE)
(72) Erfinder: FABRY, Bernd, D-41352 Korschenbroich (DE)
(86) Internationale Anmeldenummer: EP9503799
(87) Internationale Veröffentlichungsnummer: WO9610386

(56) Entgegenhaltungen:
- EP-A- 0 285 768
- EP-A- 0 450 527
- WO-A-95/17880

## Beschreibung

Die Erfindung betrifft kosmetische Mittel mit einem Gehalt an Fettsäure-N-alkylglucamiden und Siliconverbindungen.

### Stand der Technik

Fettsäure-N-alkylglucamide stellen bekannte nichtionische Tenside auf Zuckerbasis dar, die sich durch ein starkes Schaumvermögen, gute Reinigungsleistung und hohe hautkosmetische Verträglichkeit auszeichnen. Die Verwendung dieser Stoffe ist Gegenstand einer Vielzahl von Veröffentlichungen. Aus der Europäischen Patentanmeldung **EP-A1 0285768** (Hüls) ist beispielsweise ihr Einsatz als Verdickungsmittel bekannt. In der Französischen Offenlegungsschrift **FR-A 1580491** (Henkel) werden wäßrige Detergensgemische auf Basis von Sulfaten und/oder Sulfonaten, Niotensiden und gegebenenfalls Seifen beschrieben, die Fettsäure-N-alkylglucamide als Schaumregulatoren enthalten. Gegenstand der Internationalen Patentanmeldungen **WO 92/6153, WO 92/6156, WO 92/6157, WO 92/6158, WO 92/6159 und WO 92/6160** (Procter & Gamble) sind Mischungen von Fettsäure-N-alkylglucamiden mit anionischen Tensiden, Tensiden mit Sulfat- und/oder Sulfonatstruktur, Ethercarbonsäuren, Ethersulfaten, Methylestersulfonaten und nichtionischen Tensiden. Die Verwendung dieser Stoffe in den unterschiedlichsten Wasch-, Spül- und Reinigungsmitteln wird in den Internationalen Patentanmeldungen **WO 92/6152, WO 92/6154, WO 92/6155, WO 92/6161, WO 92/6162, WO 92/6154, WO 92/6170, WO 92/6171** und **WO 92/6172** (Procter & Gamble) beschrieben. Des weiteren sei auf die nachveröffentlichte **WO 95/17880** (Procter & Gamble) verwiesen, in der stark schäumende Shampoos beschrieben werden, die Mischungen von Alkyl(ether)sulfaten und Fettsäure-N-alkylglucamiden offenbaren, welche als Zusatzstoffe auch Silicone enthalten können.

Hinsichtlich der Formulierung von kosmetischen Mitteln, insbesondere Haarpflegemitteln, weisen Fettsäure-N-alkylglucamide jedoch den Nachteil auf, daß ihr Schaumvermögen nicht immer zufriedenstellend ist. Dies bezieht sich einerseits auf die Höhe des Basischaums, andererseits auch auf die Schaumbeständigkeit, insbesondere in hartem Wasser. Ein Weiterer Nachteil besteht darin, daß die Amide nach dem Abspülen oftmals ein stumpfes Gefühl auf den Haaren hinterlassen und die Kämmbarkeit tendenziell eher verschlechtern.

Die komplexe Aufgabe der Erfindung hat somit darin bestanden, kosmetische Mittel mit einem Gehalt an Fettsäure-N-alkylglucamide zur Verfügung zu stellen, die frei von den geschilderten Nachteilen sind.

### Beschreibung der Erfindung

Gegenstand der Erfindung sind kosmetische Mittel, enthaltend
(a) Fettsäure-N-alkylglucamide und
(b) Siliconverbindungen
mit der Maßgabe, daß wenn die Mittel einen Tensidgehalt von 5 bis 40 Gew.-% aufweisen und Alkylsulfate und/oder Alkylethersulfate 80 bis 99 Gew.-% dieses Tensidgehaltes ausmachen, der Anteil der Fettsäure-N-alkylglucamide Weniger als 1 oder mehr als 20 Gew.-% beträgt.

Überraschend wurde gefunden, daß der Zusatz von Siliconverbindungen das Schaumvermögen von Fettsäure-N-alkylglucamiden signifikant steigert. Die Erfindung schließt die Erkenntnis ein, daß in gleicher Weise die Schaumbeschaffenheit und die Haaravivage verbessert werden,

### Fettsäure-N-alkylglucamide

Fettsäure-N-alkylglucamide folgen der Formel **(I),** 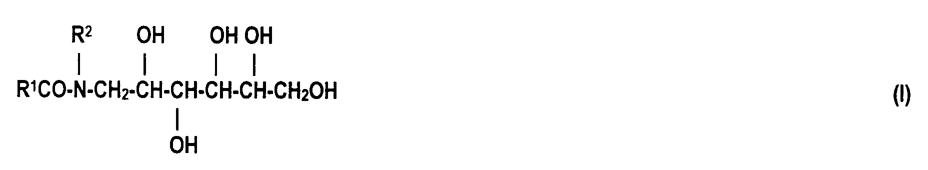 in der R¹CO für einen aliphatischen Acylrest mit 6 bis 22 Kohlenstoffatomen und R² für einen Alkyl- oder Hydroxyalkylrest mit 1 bis 4 Kohlenstoffatomen steht. Bei den Fettsäure-N-alkylglucamiden handelt es sich um bekannte Stoffe, die üblicherweise durch reduktive Aminierung von Glucose mit einem Alkylamin oder einem Alkanolamin und nachfolgende Acylierung mit einer Fettsäure, einem Fettsäurealkylester oder einem Fettsäurechlorid erhalten werden können. Hinsichtlich der Verfahren zu ihrer Herstellung sei auf die US-Patentschriften **US 1985424, US 2016962** und **US 2703798** sowie die internationale Patentanmeldung **WO 92/06984** verwiesen. Eine Übersicht zu diesem Thema von H.Kelkenberg findet sich in **Tens.Surf. Det. 25, 8 (1988)**. Schließlich sei noch auf die europäische Patentanmeldung **EP-A2 0450527** (Kao) verwiesen, die den Einsatz von Fettsäure-N-tris(hydroxymethyl)methylamiden zusammen mit Siliconverbindungen beschreibt. Vorzugsweise werden Fettsäure-N-alkylglucamide der Formel **(I)** eingesetzt, in der R² für eine Amingruppe steht und R¹CO für den Acylrest der Capronsäure, Caprylsäure, Caprinsäure, Laurinsäure, Myristinsäure, Palmitinsäure, Palmoleinsäure, Stearinsäure, Isostearinsäure, Ölsäure, Elaidinsäure, Petroselinsäure, Linolsäure, Linolensäure, Arachinsäure, Gadoleinsäure, Behensäure oder Erucasäure bzw. derer technischer Mischungen steht. Besonders bevorzugt sind Fettsäure-N-alkylglucamide der Formel **(I)**, die durch reduktive Aminierung von Glucose mit Methylamin und anschließende Acylierung mit Laurinsäure oder C_{12/14}-Kokosfettsäure bzw. einem entsprechenden Derivat erhalten werden.

### Siliconverbindungen

As Siliconverbindungen kommen beispielsweise die folgenden Verbindungen in Betracht:
(1) Dimethylpolysiloxane der Formel **(II)**,

   (CH₃)₃SiO[(CH₃)₂SiO]n₁Si(CH₃)₃ (II)

   in der n1 für eine Zahl von 3 bis 600 steht;
(2) Methylphenylpolysiloxane der Formel **(III)**, 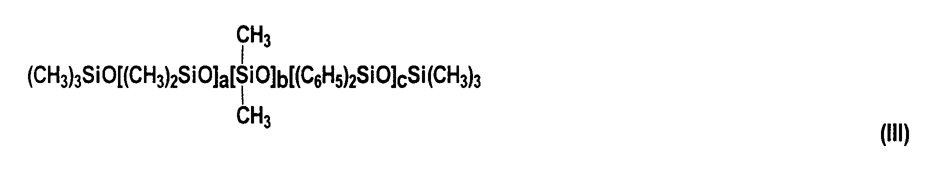 in der die Summe aus (a+b+c) für eine Zahl im Bereich von 1 bis 600 steht, mit der Maßgabe, daß mindestens zwei der drei Parameter ungleich 0 sind.
(3) Aminomodifizierte Silicone der Formel **(IV)**,

   (RdA(_{3-d})Si[OSiA)₂]ₚ-[OSi(AₑR(₂₋ₑ)]_{q}-OSi[R_{d}A_{(3-d)}] (IV)

   in der R für Wasserstoff, einen Phenylrest, eine OH-Gruppe, einen Alkylrest mit 1 bis 8 Kohlenstoffatomen, d für 0 oder eine Zahl von 1 bis 3, e für 0 oder 1, p für 0 oder Zahlen von 1 bis 1,999, q für Zahlen von 1 bis 2, die Summe (p+q) für Zahlen von 1 bis 2 und A für einen Rest C_{f}H_{2f}L steht, in dem wiederum f eine Zahl von 2 bis 8 und L eine Gruppe der Formel **(IVa)** bis **(IVf)** bedeutet: 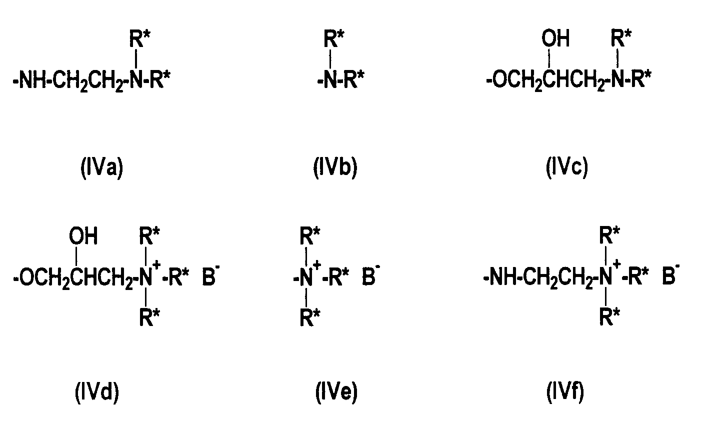
(4) Fettsäuremodifzierte Silicone der Formel **(V)**, 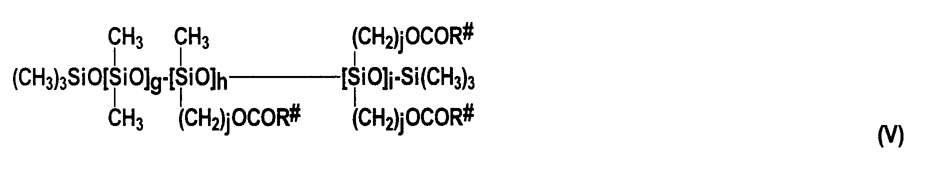 in der R#CO für einen Acylrest mit 6 bis 22 Kohlenstoffatomen, g, h und i unabhängig voneinander für Zahlen von 1 bis 350 und j für 0 oder Zahlen von 1 bis 10 stehen.
(5) Alkoholmodifizierte Silicone der Formeln **(VIa)** und/oder **(VIb)**, 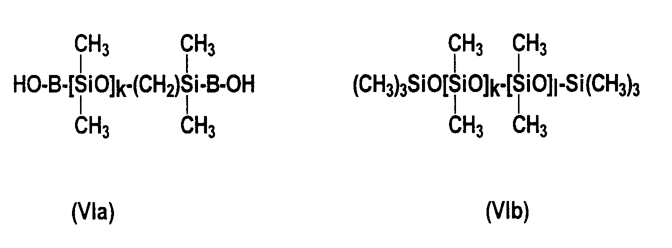 in der k und I unabhängig voneinander für Zahlen im Bereich von 1 bis 500, vorzugsweise 1 bis 200 und B für eine gegebenenfalls hydroxysubstituierte Alkylengruppe mit 1 bis 4 Kohlenstoffatomen steht.
(6) Fettsäure/Alkoholmodifzierte Silicone der Formel **(VII)**, 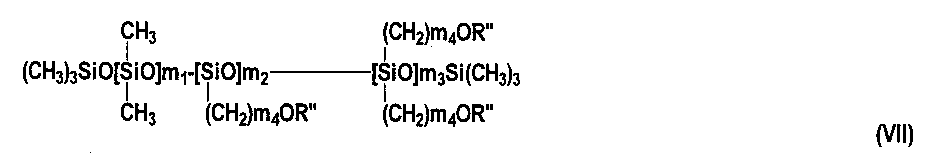 in der R'' für einen Alkylrest mit 6 bis 22 Kohlenstoffatomen, m1, m2 und m3 für Zahlen von 1 bis 300 und m4 für 0 oder Zahlen von 4 bis 22 steht.
(7) Polyethermodifzierte Silicone der Formel **(VIIIa)** und/ oder **(VIIIb)**, 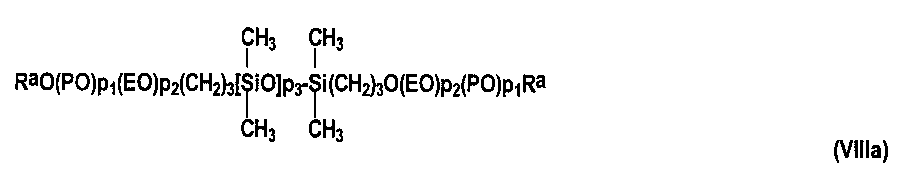 in der R^{a} für einen Alkylrest mit 1 bis 6 Kohlenstoffatomen, PO für einen OCH₂CH(CH₃)-Rest, EO für einen OCH₂CH₂-Rest, p1 und p2 unabhängig voneinander für 0 oder Zahlen von 1 bis 50 und p3 für 0 oder Zahlen von 1 bis 400 steht, sowie 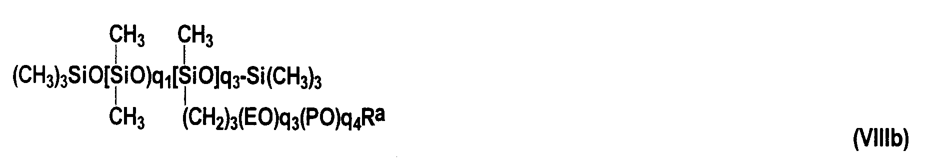 in der q1 für eine Zahl von 2 bis 100 und vorzugsweise 20 bis 80, q2 für einen Zahl von 1 bis 50 und vorzugsweise 3 bis 30, EO für einen OCH₂CH₂-Rest, PO für einen OCH₂CH(CH₃)-Rest, q3 und q4 für 0 oder Zahlen von 1 bis 50, vorzugsweise 5 bis 25 und R^{a} für einen Alkylrest mit 1 bis 6 Kohlenstoffatomen steht.
(8) Epoxymodifizierte Silicone der Formel **(IX)**, 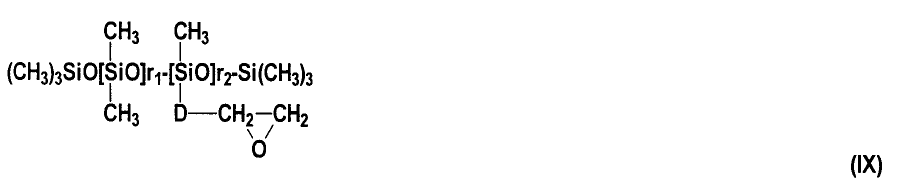 in der r1 für eine Zahl von 1 bis 500 und vorzugsweise 1 bis 250, r2 für eine Zahl von 1 bis 50 und vorzugsweise 1 bis 30 und D für eine Alkylengruppe mit 1 bis 3 Kohlenstoffatomen steht.
(9) Fluormodifizierte Silicone der Formel **(X)**, 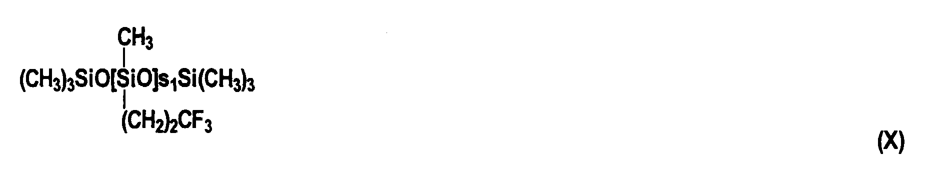 in der s1 für eine Zahl von 1 bis 400 und vorzugsweise 1 bis 250 steht.
(10) Cyclische Silicone der Formel **(XI)**, 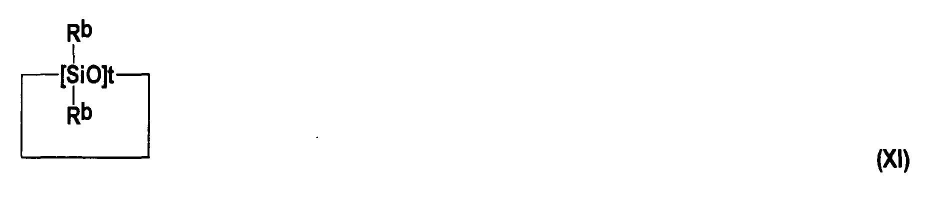 n der R^{b} für einen Alkylrest mit 1 bis 3 Kohlenstoffatomen und t für Zahlen von 3 bis 8 steht.
(11) Alkylmodifizierte Silicone der Formeln **(XIIa)** und/oder **(XIIb)**, 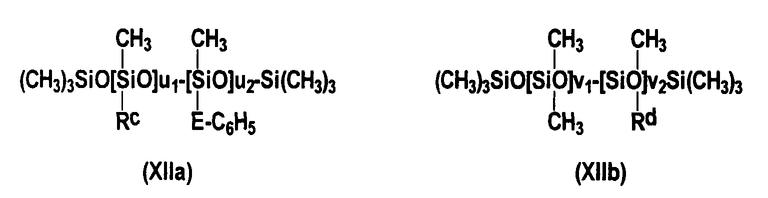 in der R^{c} für einen Alkylrest mit 2 bis 18 Kohlenstoffatomen, E für eine Alkylengruppe mit 1 bis 4 Kohlenstoffatomen und u1 und u2 unabhängig voneinander für Zahlen von 1 bis 500 und vorzugsweise 1 bis 200 stehen, sowie in der R^{d} für einen Alkylrest mit 10 bis 16 Kohlenstoffatomen und v1 und v2 unabhängig voneinander für Zahlen von 1 bis 500 und vorzugsweise 1 bis 200 stehen.

Im Hinblick auf die gewünschten Eigenschaften der oberflächenaktiven Mittel weisen bestimmte Siliconverbindungen in Kombination mit den Tensiden besondere Vorteile auf. Ein besonders feiner, cremiger Schaum wird beispielsweise mit Methylpolysiloxanen (1), Methylphenylpolysiloxanen (2), aminomodifizierten Siliconen (7), polyethermodifizierten Siliconen (7) und/oder cyclischen Siliconen (10) erzielt. Im Sinne der Erfindung kommen als Siliconverbindungen auch ausdrücklich tensidische Siliciumverbindungen in Betracht, die sich strukturell von Polydimethylsiloxanen ableiten, in denen eine oder mehrere Methylgruppen gegen hydrophile nichtionische oder ionische Reste ersetzt wurden. Übersichten zu den in diesem Zusammenhang in Betracht kommenden Stoffen sind beispielsweise von B.Grüning und G.Koerner in **Tens.Surf. Det. 26, 312 (1989)**, von P.Hameyer und C.Gould in **Manufact.Chem., 20, January 1990** sowie B.Grüning und H.Leidreiter in **Seifen- Öle-Fette-Wachse 118, 117 (1992)** erschienen. Die erfindungsgemäßen Zubereitungen können die Siliconverbindungen in Mengen von 0,01 bis 10, vorzugsweise 0,1 bis 5 und insbesondere 0,5 bis 2,5 Gew.-% - bezogen auf den Feststoffgehalt der Gemische - enthalten.

### Gewerbliche Anwendbarkeit

Mischungen von Fettsäure-N-alkylglucamiden und Siliconverbindungen sind mild und zeichnen sich durch ein verbessertes Schaumvermögen sowie eine verbesserte Haaravivage aus. Sie eignen sich daher zur Herstellung von kosmetischen Mitteln, insbesondere haarkosmetischen Mitteln wie beispielsweise Haarshampoos, Haarspülungen und Haarkuren.

### Kosmetische Mittel

Unter kosmetischen Mitteln sind im Sinne der Erfindung vorzugsweise Haut- und Haarpflegemittel zu verstehen. Diese Mittel können in untergeordneten Mengen weitere, mit den anderen Inhaltsstoffen kompatible **Tenside** enthalten. Typische Beispiele sind Fettalkoholpolyglycolethersulfate, Monoglyceridsulfate, Ethercarbonsäuren, Mono- und/oder Dialkylsulfosuccinate, Fettsäureisethionate, Fettsäuresarcosinate Fettsäuretauride, Alkyl- und/oder Alkenyloligoglucoside, Alkylamidobetaine und/oder Proteinhydrolysate bzw. deren Kondensate mit Fettsäuren auf tierischer oder vorzugsweise pflanzlicher Basis.

**Hautpflegemittel,** wie Cremes, Lotionen und dergleichen, weisen in der Regel - neben den bereits genannten Tensiden - einen Gehalt an Ölkörpern, Emulgatoren, Fetten und Wachsen, Stabilisatoren sowie ebenfalls Überfettungsmitteln, Verdikkungsmitteln, biogenen Wirk- stoffen, Filmbildnem, Konservierungsmitteln, Farb- und Duftstoffen auf. **Haarpflegemittel**, wie beispielsweise Haarshampoos, Haarlotionen, Schaumbäder und dergleichen, können als weitere Hilfs- und Zusatzstoffe - neben den bereits genannten Tensiden - Emulgatoren, Überfettungsmittel, Verdickungsmittel, biogene Wirkstoffe, Filmbildner, Konservierungsmittel, Farb- und Duftstoffe enthalten.

Als Ölkörper kommen beispielsweise Guerbetalkohole auf Basis von Fettalkoholen mit 6 bis 18, vorzugsweise 8 bis 10 Kohlenstoffatomen, Ester von linearen C₆-C₂₀-Fettsäuren mit linearen C₆-C₂₀-Fettalkoholen, Ester von verzweigten C₆-C₁₃-Carbonsäuren mit linearen C₁₆-C₁₈-Fettalkoholen, Ester von linearen C₁₀-C₁₈-Fettsäuren mit verzweigten Alkoholen, insbesondere 2-Ethylhexanol, Ester von linearen und/oder verzweigten Fettsäuren mit zweiwertigen Alkoholen und/oder Guerbetalkoholen, Triglyceride auf Basis C₆-C₁₀-Fettsäuren, pflanzliche Öle, verzweigte primäre Alkohole, substituierte Cyclohexane, Guerbetcarbonate und/oder Dialkylether in Betracht. Als **Emulgatoren** kommen sowohl bekannte W/O- als auch O/W- Emulgatoren wie beispielsweise gehärtetes und ethoxyliertes Ricinusöl, Polyglycerinfettsäureester oder Polyglycerinpolyricinoleate in Frage. Typische Beispiele für **Fette** sind Glyceride, als **Wachse** kommen u.a. Bienenwachs, Paraffinwachs oder Mikrowachse gegebenenfalls in Kombination mit hydrophilen Wachsen, z.B. Cetylstearylalkohol in Frage. Als **Stabilisatoren** können Metallsalze von Fettsäuren wie z.B. Magnesium-, Aluminium- und/oder Zinkstearat eingesetzt werden. As **Überfettungsmittel** können Substanzen wie beispielsweise polyethoxylierte Lanolinderivate, Lecithinderivate, Polyolfettsäureester, Monoglyceride und Fettsäurealkanolamide verwendet werden, wobei die letzteren gleichzeitig als Schaumstabilisatoren dienen. Geeignete **Verdickungsmittel** sind beispielsweise Polysaccharide, insbesondere Xanthan-Gum, Guar-Guar, Agar-Agar, Alginate und Tylosen, Carboxymethylcellulose und Hydroxyethylcellulose, ferner höhermolekulare Polyethylenglycolmono- und -diester von Fettsäuren, Polyacrylate, Polyvinylalkohol und Polyvinylpyrrolidon, Tenside wie beispielsweise Fettalkoholethoxylate mit eingeengter Homologenverteilung oder Alkyloligoglucoside sowie Elektrolyte wie Kochsalz und Ammoniumchlorid. Unter **biogenen Wirkstoffen** sind beispielsweise Pflanzenextrakte und Vitaminkomplexe zu verstehen. Gebräuchliche **Filmbildner** sind beispielsweise Chitosan, mikrokristallines Chitosan, quaterniertes Chitosan, Polyvinylpyrrolidon, Vinylpyrrolidon-Vinylacetat-Copolymerisate, Polymere der Acrylsäurereihe, quaternäre Cellulose-Derivate und ähnliche Verbindungen. Als **kationische Polymere** kommen kationische Cellulosederivate, kationische Stärken, Copolymere von Diallylammoniumsalzen und Acrylamiden, quaternierte Polyvinylpyrrolidon-Derivate, quaternierte Vinylpyrrolidon Vinylimidazol-Polymere, Kondensationsprodukte von Polyglycolen und Aminen, quaternierte Kollagenpolypeptide, Polyethylenimin, Copolymere der Adipinsäure und Dimethylaminohydroxypropyldiethylentrimamin, Polyaminopolyamide, kationischen Chitinderivate, kationische Guar-Gum und/oder quaternierten Ammoniumsalz-Polymere in Betracht. Als **Konservierungsmittel** eignen sich beispielsweise Phenoxyethanol, Formaldehydlösung, Parabene, Pentandiol oder Sorbinsäure. Als **Perlglanzmittel** kommen beispielsweise Glycoldistearinsäureester wie Ethylenglycoldistearat, aber auch Fettsäuremonoglycolester in Betracht. Als **Farbstoffe** können die für kosmetische Zwecke geeigneten und zugelassenen Substanzen verwendet werden, wie sie beispielsweise in der Publikation **"Kosmetische Färbemittel" der Farbstoffkommission der Deutschen Forschungsgemeinschaft, veröffentlicht im Verlag Chemie, Weinheim, 1984, S.81-106** zusammengestellt sind. Diese Farbstoffe werden üblicherweise in Konzentrationen von 0,001 bis 0,1 Gew.-%, bezogen auf die gesamte Mischung, eingesetzt.

Der Gesamtanteil der Hilfs- und Zusatzstoffe kann 1 bis 50, vorzugsweise 5 bis 40 Gew.-% und der nicht wäßrige Anteil ("Aktivsubstanzgehalt") 20 bis 80, vorzugsweise 30 bis 70 Gew.-% - bezogen auf die Mittel - betragen. Die Herstellung der Mittel kann in an sich bekannter Weise, d.h. beispielsweise durch Heiß-, Kalt-, Heiß-Heiß/Kalt- bzw. PIT-Emulgierung erfolgen. Hierbei handelt es sich um en rein mechanisches Verfahren, eine chemische Reaktion findet nicht statt.

### Beispiele

**Schaumvermögen**. Das Schaumvermögen verschiedener Rezepturen wurde in hartem Wasser (16 d) bei 23°C gemäß DIN 53 902/Teil 2 bestimmt. Angegeben sind der Basisschaum, die Schaumhöhe nach 5 min, die Beschaffenheit des Schaums (++ = cremiger, ergiebiger Schaum; + = grobporiger Schaum) sowie das subjektive Haargefühl bei Anwendung als Haarshampoo nach dem Ausspülen (+++ glatt, leicht kämmbar, ++ stumpf, leicht kämmbar, + stumpf, schwer kämmbar. Die Beispiele 1 bis 4 sind erfindungsgemäß, die Beispiele V1 bis V4 dienen zum Vergleich. Die Ergebnisse sind in Tabelle 1 zusammengefaßt.

**Tabelle 1**

| Schaumvermögen (Mengenangaben als Gew.-%) | | | | | | | | |
|---|---|---|---|---|---|---|---|---|
| **Zusammensetzung/Performance** | **1** | **2** | **3** | **4** | **V1** | **V2** | **V3** | **V4** |
| C_{12/14}-Kokosalkyl-N-methylglucamid | 4,0 | - | 2,0 | - | 4,0 | - | - | |
| Lauryl-N-methylglucamid | - | 4,0 | - | 2,0 | - | 2,0 | - | - |
| C_{12/14}-Kokosalkohol+3,6EO-sulfat-Na-Salz | 8,0 | 8,0 | 6,0 | 6,0 | 8,0 | 6,0 | 8,0 | 6,0 |
| C_{8/10}-Alkylpolyglucosid, DP=1,5 | - | - | - | - | - | - | 4,0 | 2,0 |
| Sojaprotein-Kokosfett-Säurekondensat | - | - | 2,0 | 2,0 | - | 2,0 | - | 2,0 |
| Betaine auf Basis Kokosfettsäure | - | - | 2.0 | 2.0 | - | 2.0 | - | 2.0 |
| Dimethylpolysiloxan (Th.Goldschmidt) | 1,0 | - | - | - | - | - | 1,0 | - |
| Methylphenylpolysiloxan (KF56, Shin-Etsu) | - | 1,0 | - | - | - | - | - | - |
| Aminomodifiziertes Siloxan (SF8517, Toray) | - | - | 1,0 | - | - | - | - | - |
| Aminoalkylpolysiloxan (SM8702, Toray) | - | - | - | 1,0 | - | - | - | 1,0 |
| 2-Octyldodecanol | 2,0 | - | - | - | 2,0 | - | 2,0 | - |
| Dioctylcyclohexane | - | 2,0 | - | - | - | - | - | 2,0 |
| Almond Oil | - | - | 2,0 | - | - | 2,0 | - | - |
| Di-n-octylether | - | - | - | 2,0 | - | - | - | - |
| Cetearyl Alcohol | 3,0 | 3,0 | 3,0 | 3,0 | 3,0 | 3,0 | 3,0 | 3,0 |
| Wasser | ad 100 | | | | | | | |
| ***Schaumvermögen*** | | | | | | | | |
| **- Basisschaum** | 650 | 600 | 680 | 530 | 530 | 590 | 580 | 600 |
| ***-Schaumhöhe nach 5 min*** | 600 | 580 | 610 | 480 | 470 | 500 | 500 | 510 |
| ***Schaumperformance*** | ++ | ++ | ++ | ++ | + | + | ++ | ++ |
| ***Subjektives Haargefühl nach Ausspülen*** | +++ | +++ | +++ | +++ | ++ | ++ | + | + |

**Trocken- und Naßkämmbarkeitsuntersuchungen.** Die Trockenkammbarkeit wurde unter Zulassung der elektrostatischen Aufladung untersucht. Es wurde eine relative Luftleuchtigkeit von 20 % eingestellt. Die Konditionierungszeit betrug 12 h bei 30°C. Die Messung erfolgte Ober den Ladungsabgriff an einem doppelten Faraday-Käfig nach Ausführung von 10 Kämmungen. Der Fehler bei den Messungen betrug im Mittel 2,5 %, die statistische Sicherheit lag bei mindestens 99,9 %. Die Naßkämmbarkeit wurde an braunem Haar (Alkinco #6634, Strähnenlänge 12 cm, Strähnenmasse 1 g) untersucht. Nach der Nullmessung wurden die Strähnen mit 100 ml der Formulierungen 1 bis 4 bzw. V1 bis V4 getränkt. Nach einer Einwirkzeit von 5 min wurden die Strähnen 1 min unter fließendem Wasser (1 l/min, 38°C) ausgespült. Die Strähnen wurden erneut vermessen und mit der Nullmessung verglichen. Der Fehler bei den Messungen betrug im Mittel 2 %, die statistische Sicherheit lag bei mindestens 99 %. Die Ergebnisse sind in Tabelle 2 dargestellt.

**Tabelle 2**

| Trocken- und Nasskämmbarkeit | | | | | | | | |
|---|---|---|---|---|---|---|---|---|
| **Performance** | **1** | **2** | **3** | **4** | **V1** | **V2** | **V3** | **V4** |
| | | | | | | | | |

| ***Trockenkämmbarkeit [mJ]*** | | | | | | | | |
|---|---|---|---|---|---|---|---|---|
| ***- vorher*** | 4,8 | 4,9 | 5,3 | 4,8 | 4,1 | 4,3 | 4,5 | 4,5 |
| ***-nachher*** | 4,0 | 4,3 | 4,2 | 4,0 | 3,9 | 4,2 | 4,1 | 4,1 |
| ***- Differenz*** | 0,8 | 0,6 | 1,1 | 0,8 | 0,2 | 0,1 | 0,4 | 0,4 |

| ***Naßkämmbarkeit [mJ]*** | | | | | | | | |
|---|---|---|---|---|---|---|---|---|
| ***- vorher*** | 65,9 | 65,7 | 86,9 | 75,5 | 57,2 | 54,0 | 58,8 | 62,0 |
| ***-nachher*** | 20,1 | 21,5 | 27,5 | 23,4 | 27,8 | 24,9 | 21,0 | 25,8 |
| ***- Differenz*** | 45,8 | 44,2 | 62,1 | 52,1 | 29,4 | 29,1 | 37,8 | 36,2 |

## Patentansprüche

1. Kosmetische Mittel, enthaltend
(a) Fettsäure-N-alkylglucamide und
(b) Siliconverbindungen
mit der Maßgabe, daß wenn die Mittel einen Tensidgehalt von 5 bis 40 Gew.-% aufweisen und Alkylsulfate und/oder Alkylethersulfate 80 bis 99 Gew.-% dieses Tensidgehaltes ausmachen, der Anteil der Fettsäure-N-alkylglucamide weniger als 1 oder mehr als 20 Gew.-% beträgt.

2. Kosmetische Mittel nach Anspruch 1, **dadurch gekennzeichnet**, daß sie die Fettsäure-N-alkylglucamide in Mengen von 0,5 bis 25 Gew.-% - bezogen auf die Mittel - enthalten.

3. Kosmetische Mittel nach den Ansprüchen 1 oder 2, **dadurch gekennzeichnet**, daß sie Siliconverbindungen enthalten, die ausgewählt sind aus der Gruppe, die gebildet wird von Dimethylpolysiloxanen, Methylphenylpolysiloxanen, aminomodifzierten Siliconen, fettsäuremodifizierten Siliconen, alkoholmodifizierten Siliconen, fettsäure/alkoholmodifizierten Siliconen, polyethermodifizierten Siliconen, epoxymodifizierten Siliconen, fluormodifizierten Siliconen, cyclischen Siliconen und alkylmodifizierten Siliconen.

4. Kosmetische Mittel nach mindestens einem der Ansprüche 1 bis 3, **dadurch gekennzeichnet**, daß sie die Siliconverbindungen in Mengen von 0,01 bis 10 Gew.-% enthalten.

5. Kosmetische Mittel nach mindestens einem der Ansprüche 1 bis 4, **dadurch gekennzeichnet**, daß sie weiterhin kationische Polymere enthalten.

## Claims

1. Cosmetic compositions containing
(a) fatty acid-N-alkyl glucamides and
(b) silicone compounds,
with the proviso that, when the compositions have a surfactant content of 5 to 40% by weight and alkyl sulfates and/or alkyl ether sulfates make up 80 to 99% by weight of that surfactant content, the percentage fatty acid-N-alkyl glucamide content is less than 1 or more than 20% by weight.

2. Cosmetic compositions as claimed in claim 1, characterized in that they contain the fatty acid-N-alkyl glucamides in quantities of 0.5 to 25% by weight, based on the composition.

3. Cosmetic compositions as claimed in claim 1 or 2, characterized in that they contain silicone compounds selected from the group consisting of dimethyl polysiloxanes, methylphenyl polysiloxanes, aminomodified silicones, fatty-acid-modified silicones, alcohol-modified silicones, fatty acid/alcohol-modified silicones, polyether-modified silicones, epoxy-modified silicones, fluorine-modified silicones, cyclic silicones and alkyl-modified silicones.

4. Cosmetic compositions as claimed in at least one of claims 1 to 3, characterized in that they contain the silicone compounds in quantities of 0.01 to 10% by weight.

5. Cosmetic compositions as claimed in at least one of claims 1 to 4, characterized in that they additionally contain cationic polymers.

## Revendications

1. Produits cosmétiques contenant :
a) des N-alkylglucamides d'acide gras et,
b) des composés de silicone,
avec la restriction que lorsque les produits possèdent une teneur en agent tensioactif allant de 5 à 40 % en poids et que les alkylsulfates et/ou les alkyléthersulfates représentent de 80 à 99 % en poids de cette teneur en agent tensioactif, la proportion de N-alkylglucamide d'acide gras s'élève à moins de 1 ou à plus de 20 % en poids.

2. Produits cosmétiques selon la revendication 1,
caractérisés en ce qu' ils renferment les N-alkylglucamides d'acide gras en quantités allant de 0,5 à 25 % en poids rapporté au produit.

3. Produits cosmétiques selon les revendications 1 ou 2, caractérisés en ce qu' ils renferment des composés de silicone qui sont choisis dans le groupe formé des diméthylpolysiloxanes, des méthylphénylpolysiloxanes, des silicones modifiées par une amine, des silicones modifiées par des acides gras, des silicones modifiées par un alcool, des silicones modifiées par un acide gras/un alcool, des silicones modifiées par un polyéther, des silicones modifiées par un époxy, des silicones modifiées par du fluor, des silicones cycliques et des silicones modifiées par un alkyle.

4. Produits cosmétiques selon au moins une des revendications 1 à 3,
caractérisés en ce qu' ils renferment les composés de silicone en quantités allant de 0,01 à 10 % en poids.

5. Produits cosmétiques selon au moins une des revendications 1 à 4,
caractérisés en ce qu' ils renferment en outre des polymères cationiques.
